(19) Europäisches Patentamt
European Patent Office
Office européen des brevets

(11) Veröffentlichungsnummer: **0 005 154**
**B1**

# (12) EUROPÄISCHE PATENTSCHRIFT

(45) Veröffentlichungstag der Patentschrift: **25.11.81**

(21) Anmeldenummer: **79100676.0**

(22) Anmeldetag: **07.03.79**

(51) Int. Cl.³: **C 07 D 513/04,** C 07 D 513/14, A 61 K 31/505, C 07 D 239/54, C 07 D 405/04 // (C07D513/04, 277/00, 239/00),(C07D513/14, 317/00, 277/00, 239/00)

(54) **1-Oxo-1H-pyrimido (6,1-b) benzthiazol-Derivate, Verfahren zu deren Herstellung sowie diese Verbindungen enthaltende Arzneimittel.**

(30) Priorität: **13.03.78 DE 2810863**

(43) Veröffentlichungstag der Anmeldung: **14.11.79 Patentblatt 79/23**

(45) Bekanntmachung des Hinweises auf die Patenterteilung: **25.11.81 Patentblatt 81/47**

(84) Benannte Vertragsstaaten: **BE CH DE FR GB IT LU NL SE**

(56) Entgegenhaltungen:
**DE-A-1 670 484**
**US-A-4 041 163**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Winter, Werner, Dr., Mannheimer Strasse 9, D-6148 Heppenheim (DE)**
Erfinder: **Hindermayr, Herman, Dr., Geraer Ring 2/90, D-6800 Mannheim 42 (DE)**
Erfinder: **Roesch, Egon, Dr., Am Oberen Luisenpark 22, D-6800 Mannheim 1 (DE)**
Erfinder: **Roesch, Androniki, Dr., Am Oberen Luisenpark 22, D-6800 Mannheim 1 (DE)**
Erfinder: **Wilhelms, Otto-Henning, Dr., Odenwaldstrasse 25/2, D-6941 Weinheim-Rittenweier (DE)**

EP 0 005 154 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1-Oxo-1H-pyrimido(6,1-b)benzthiazol-Derivate, Verfahren zu deren Herstellung sowie diese Verbindungen enthaltende Arzneimittel

Die vorliegende Erfindung betrifft neue 1-Oxo-1H-pyrimido(6,1-b)benzthiazol-Derivate, Verfahren zu deren Herstellung sowie diese Verbindungen enthaltende Arzneimittel.

Die neuen Verbindungen sowie ihre pharmakologisch verträglichen Salze weisen bei parenteraler und auch peroraler Gabe eine ausgeprägte antiallergische Wirkung auf und können daher besonders vorteilhaft bei der Bekämpfung von allergischen Krankheiten, beispielsweise von allergischem Asthma, Heuschnupfen und Urticaria, verwendet werden. Sie besitzen ferner antiödematöse und antiphlogistische Eigenschaften.

Die vorliegende Erfindung betrifft 1-Oxo-1H-pyrimido(6,1-b)benzthiazol-Derivate der allgemeinen Formel I

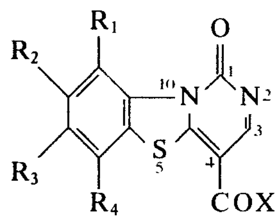

(I)

in der

$R_1$, $R_2$, $R_3$ und $R_4$ jeweils Wasserstoff oder Halogen, eine Hydroxy-, Nitro- oder Trifluormethyl-Gruppe, einen niederen geradkettigen oder verzweigten Alkyl-, Alkoxy- oder Alkylthio-Rest bedeuten, wobei $R_2$ und $R_3$ zusammen auch eine Alkylendioxy-Gruppe vorstellen können, und
X eine Hydroxy-, Alkoxy- oder Tetrazolyl-5-amino-Gruppe bedeutet,

und deren pharmakologisch verträgliche Salze, Verfahren zur Herstellung dieser Verbindungen sowie ihre Verwendung bei der Bekämpfung von allergischen Krankheitsbildern.

Weiterhin betrifft die Erfindung pharmazeutische Präparate mit einem Gehalt an Verbindungen der allgemeinen Formel I zur Herstellung solcher Präparate, wobei für den Fall, daß X eine Hydroxygruppe bedeutet, die Verbindungen der Formel I gegebenenfalls in der Form von anorganischen Salzen, vorzugsweise von Alkalimetall-Salzen oder von organischen Salzen, vorzugsweise von primären, sekundären oder tertiären organischen Aminen, Anwendung finden.

Unter einem niederen, geradkettigen oder verzweigten Alkyl-, Alkoxy- oder Alkylthio-Rest in der Definition der Substituenten $R_1$, $R_2$, $R_3$, $R_4$ und X ist ein Rest mit 1 bis 6, vorzugsweise 1 bis 4 Kohlenstoffatomen zu verstehen, besonders bevorzugt ist die Methyl-, Ethyl-, n-Propyl-, Isopropyl- oder tert. Butylgruppe sowie die entsprechenden Alkoxy- und Alkylthio-Reste.

Die Alkylendioxygruppe der Substituenten $R_2$ und $R_3$ soll vorzugsweise 1 bis 3 Kohlenstoffatome enthalten, wobei die Methylendioxygruppe besonders bevorzugt ist.

Unter Halogen ist Fluor, Chlor und Brom zu verstehen, vorzugsweise Chlor.

Die neuen 1-Oxo-1H-pyrimido(6,1-b)benzthiazol-Derivate der Formel I weisen im Vergleich zum nächstliegenden Stand der Technik bessere antiallergische, antiödematöse und antiphlogistische Eigenschaften auf.

Zum Beweis wurden im pharmakologischen Test die passive cutane anaphylaktische Reaktion (PCA-Werte) in vivo bei Ratten für das Handelsprodukt HETRAZAN (Diethylcarbamazin; 1-Diethylcarbamoyl-4-methylpiperazin) und für die Verbindung des Beispiels 5a) (7-Ethoxy-1-oxo-1H-pyrimido(6,1-b)benzthiazol-4-carbonsäure) ermittelt. Mit der erfindungsgemäßen Verbindung läßt sich selbst bei zwanzigfach geringerer Dosis eine achtmal bessere Inhibierung der passiven cutanen anaphylaktischen Reaktionen als mit dem Handelsprodukt erreichen. Anhand einer geeigneten Auswahl aus den beanspruchten Substanzen konnte aufgezeigt werden, daß auch die anderen erfindungsgemäßen Verbindungen eine ebenso gute, teilweise sogar noch bessere Inhibierung der passiven cutanen anaphylaktischen Reaktion bewirken.

Die Hemmpotenz der beanspruchten Substanzklasse kann auch in vitro anhand der Antigen-induzierten Mastzellendegranulation überzeugend dargestellt werden.

Die neuen Verbindungen der allgemeinen Formel I lassen sich auf verschiedene Weise zu Substanzen weiterverarbeiten, die ebenfalls pharmakologische Wirksamkeit aufweisen. Sie stellen daher auch wertvolle Zwischenprodukte zur Herstellung von pharmakologisch wirksamen Stoffen dar.

Das erfindungsgemäße Verfahren zur Herstellung der Verbindungen der allgemeinen Formel I

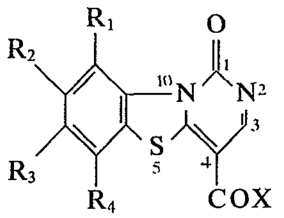

(I)

in der

$R_1$, $R_2$, $R_3$, $R_4$ und X die oben angegebene Bedeutung haben,

und deren pharmakologisch verträglichen Salze,
ist dadurch gekennzeichnet, daß man ein 4-Oxo-4H-pyrimido(2,1)benzthiazol der allgemeinen Formel II

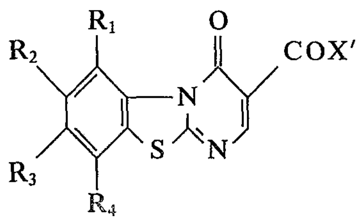

(II)

in der

$R_1$, $R_2$, $R_3$, $R_4$ die oben angegebene Bdeutung haben und X′ einen niederen Alkoxyrest oder eine Hydroxygruppe vorstellt,

mit überschüssiger Base zu den in Lösung im tautomeren Gleichgewicht stehenden 1-(2-Mercapto-phenyl)-pyrimidin-2,6-dionen der allgemeinen Formel III A—IIII C

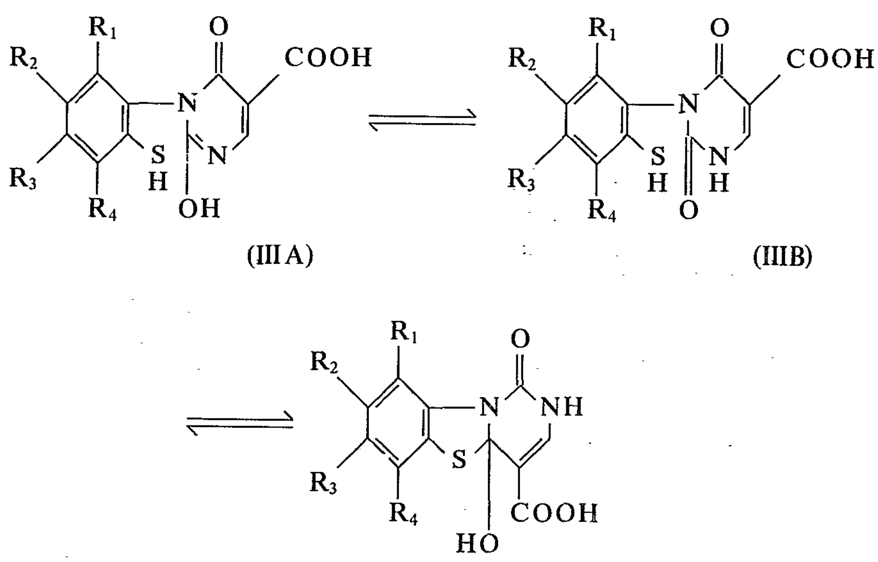

(IIIA)

(IIIB)

(IIIC)

in denen

$R_1$, $R_2$, $R_3$ und $R_4$ die oben angegebene Bedeutung haben,
bzw. deren Alkalisalzen hydrolysiert und diese unter sauren Reaktionsbedingungen durch Wasserabspaltung zu erfindungsgemäßen Verbindungen der allgemeinen Formel I, in der X eine Hydroxygruppe bedeutet, cyclisiert,
wobei man gegebenenfalls anschließend in beliebiger Reihenfolge
die erhaltenen Carbonsäuren in an sich bekannter Weise in deren Ester oder Tetrazolyl 5 amide der allgemeinen Formel I, in der X eine Alkoxy- oder Tetrazolyl-5-amino-Gruppe vorstellt, überführt,

für den Fall, daß in Verbindungen der allgemeinen Formel I einer der Reste $R_1$, $R_2$, $R_3$ und $R_4$ eine Nitrogruppe bedeutet, diese gegebenenfalls nachträglich einführt,
einen bestimmten Substituenten $R_1$, $R_2$, $R_3$, $R_4$ oder X in einen anderen Substituenten $R_1$, $R_2$, $R_3$, $R_4$ oder X umwandelt
und/oder die erhaltenen Carbonsäuren bzw. Tetrazolylamide der Formel I in pharmakologisch verträgliche Salze überführt.

Die Ausgangsverbindungen der allgemeinen Formel II sind bekannt (vgl. z. B. D. W. Dunwell, D. Evans, J. Chem. Soc. (C), 2094 (1971)) oder lassen sich in Analogie zu den bekannten Verfahren herstellen. So werden 2-Amino-benzthiazole der allgemeinen Formel IV

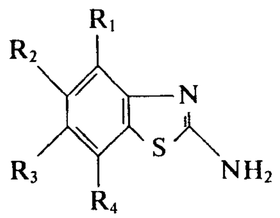
(IV)

in der

$R_1$, $R_2$, $R_3$ und $R_4$ die oben angegebene Bedeutung haben,

mit Ethoxymethylenmalonester, vorzugsweise mit Ethoxymethylenmalonsäurediethylester, zu Aminomethylen-Derivate der allgemeinen Formel V

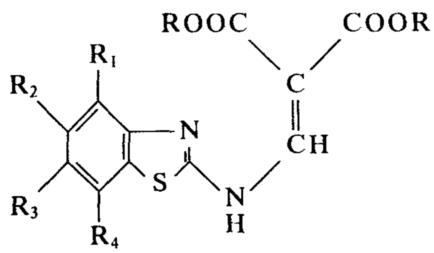
(V)

in der

$R_1$, $R_2$, $R_3$ und $R_4$ die oben angegebene Bedeutung haben und
R eine niedere Alkylgruppe, vorzugsweise Methyl oder Ethyl, vorstellt,

umgesetzt, die sich in bekannter Weise zu den Carbonsäureestern der allgemeinen Formel II (X' = Alkoxy) cyclisieren lassen. Nach Alaimo (J. Het. Chem. 10, 769 [1973]) gelangt man von diesen Carbonsäureestern der allgemeinen Formel II (X' = Alkoxy) zu den Carbonsäuren der allgemeinen Formel II (X' = Hydroxy) durch saure oder alkalische Verseifung.

Es wurde nun überraschenderweise gefunden, daß bei den Carbonsäuren sowie den Carbonsäureestern der allgemeinen Formel II, wenn diese mit überschüssigem Alkali verseift und anschließend wieder mit einer Säure behandelt werden, nicht mehr das ursprüngliche Ringsystem der allgemeinen Formel II rückgebildet wird, sondern eine Umlagerung zu dem neuen Ringsystem der erfindungsgemäßen Verbindungen der allgemeinen Formel I erfolgt.

Das erfindungsgemäße Verfahren wird vorteilhaft so durchgeführt, daß man Verbindungen der allgemeinen Formel II mit einer Base im Überschuß, vorzugsweise mit Natron- oder Kalilauge, der ein geeignetes organisches, mit Wasser mischbares Lösungsmittel, beispielsweise Methanol oder Ethanol, zugegeben werden kann, versetzt und das erhaltene Reaktionsgemisch ungefähr 0,5 bis 2 Stunden erhitzt. Durch Spaltung der einen Schwefel-Kohlenstoffbindung des Benzthiazols entstehen die neuen, im tautomeren Gleichgewicht befindlichen Zwischenprodukte der allgemeinen Formel IIIA bis IIIC. Durch vorsichtiges Neutralisieren der Reaktionslösung mit einer geeigneten Säure, vorzugsweise verdünnte Salzsäure, läßt sich das Zwischenprodukt der Formel IIIB ohne Schwierigkeiten fassen.

Die Cyclisierung der Verbindungen der allgemeinen Formel III erfordert ein stark saures, wasserabspaltendes Reaktionsmedium, das auf verschiedenste Weise erreicht werden kann. Beispielsweise kann man die Pyrimidindione der Formel III mit Polyphosphorsäure oder Polyphosphorsäureester erhitzen, vorzugsweise auf ungefähr 120°C. Die Cyclisierung läßt sich auch gut durch Erhitzen in Dioxan nach Zusatz dioxanischer Salzsäure oder in 20%iger bzw. konzentrierter Salzsäure durchführen. Ganz besonders vorteilhaft ist die Verwendung eines stark sauren Ionenaustauschers, beispielsweise die Amberlite® IRC 120 oder Amberlyst 15, in Gegenwart eines geeigneten Lösungsmittels, wie Dioxan oder Dimethylformamid, bei Raumtemperatur oder

gegebenenfalls bei erhöhter Temperatur.

Als stark saures, wasserabspaltendes Mittel kann auch ein Bortrifluorid-Essigsäure-Komplex oder auch eine Lewis-Säure, z. B. Aluminiumtrichlorid, in einem organischen Lösungsmittel, beispielsweise Chloroform, eingesetzt werden.

Wenn Verbindungen der allgemeinen Formel III in alkoholischer Salzsäure unter Rückfluß erhitzt werden, findet neben dem Ringschluß auch eine Veresterung zu Carbonsäureestern der allgemeinen Formel I mit X = Alkoxy statt.

Als vereinfachte Variante des erfindungsgemäßen Verfahrens kann in manchen Fällen auf die Isolierung der Zwischenprodukte der allgemeinen Formel III verzichtet werden. Hierzu wird unmittelbar nach der alkalischen Spaltung der Verbindungen der allgemeinen Formel II durch Zugabe eines Überschusses einer Mineralsäure der Ringschluß zu den erfindungsgemäßen Verbindungen der Formel I vollzogen.

Es wurde weiterhin überraschend gefunden, daß man auch von den bekannten Aminomethylen-Verbindungen V direkt zu den erfindungsgemäßen Verbindungen I gelangen kann, wenn man erstere unter den gleichen alkalischen Bedingungen, wie oben für die Ausgangsprodukte der allgemeinen Formel II gefordert, behandelt. Es entstehen dabei die Zwischenprodukte der allgemeinen Formel III, die dann in der oben beschriebenen Weise zu den Verbindungen der Formel I weiterverarbeitet werden.

Eine ganz besonders vorteilhafte Variante zur Durchführung des erfindungsgemäßen Verfahrens besteht darin, daß man in an sich bekannter Weise 2-Aminobenzthiazole IV mit Ethoxymethylenmalonsäureestern in einem geeigneten Lösungsmittel zu den Enaminen V, bei vorzugsweise 100—120°C kondensiert und danach bei erhöhter Temperatur, vorzugsweise 200—240°C, zu den 4-Oxo-4H-pyrimido(2,1-b)benzthiazolen der Formel II cyclisiert, anschließend, ohne die Verbindungen der Formel II zu isolieren, die alkalische Spaltung in einem Zweiphasen-Reaktionsgemisch unter Bildung der Zwischenprodukte III vornimmt und diese zu den gewünschten Endprodukten der Formel I weiterverarbeitet.

Diese Verfahrensvariante läßt sich in vorteilhafter Weise derart modifizieren, daß das für den Ringschluß der Verbindungen V geeignete Lösungsmittel nach erfolgter Cyclisierung weitgehend verdampft, und anschließend, ohne die Pyrimido(2,1-b)benzthiazole der Formel II zu isolieren, die alkalische Spaltung in einem Einphasen-Reaktionsgemisch, vorzugsweise alkoholische Alkali-Lauge, unter Bildung der Zwischenprodukte III durchgeführt wird.

Die Veresterung einer nach einer der vorstehenden Varianten erhaltenen Carbonsäuren der allgemeinen Formel I, wird zweckmäßig in Gegenwart eines sauren Katalysators, wie z. B. Chlorwasserstoff, Schwefelsäure, p-Toluolsulfosäure oder eines stark sauren Ionenaustauscherharzes, vorgenommen. Man kann auch ein Alkalisalz der Carbonsäure oder deren Salz mit einer organischen Base mit einem Alkylhalogenid in einem geeigneten Lösungsmittel, wie beispielsweise Hexamethylphosphorsäuretriamid, umsetzen. Umesterungen hingegen erfordern meistens den Zusatz einer geringen Menge einer basischen Substanz, z. B. eines Alkali- oder Erdalkalihydroxids oder eines Alkalialkoholats.

Für die Kondensation einer Carbonsäure der allgemeinen Formel I mit 5-Amino-tetrazol benutzt man die in der Literatur bekanntgewordenen Methoden der Amidierung, wobei vorzugsweise 1,1'-Carbonyl-diimidazol oder Dicyclohexyl-carbodiimid zur Anwendung kommen. Man kann aber auch in bekannter Weise die Carboxyl-Gruppe in ein rekationsfähiges Derivat, beispielsweise in ein Säurehalogenid, einen aktiven Ester oder gemischtes Anhydrid, überführen und dieses mit 5-Amino-tetrazol umsetzen.

Zur Herstellung von Salzen mit pharmakologisch verträglichen organischen oder anorganischen Basen, wie z. B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen der allgemeinen Formel I auch in Form von Pudern und Salben verwendet werden, sie werden dazu z. B.

mit pulverförmigen, physiologisch verträglichen Verdünnungsmitteln bzw. üblichen Salbengrundlagen vermischt.

Außer den in den nachstehenden Beispielen genannten Substanzen sind im Sinne der vorliegenden Anmeldung die folgenden Verbindungen bevorzugt:

7-t-Butyl-1-oxo-1H-pyrimido[6,1-b]benzthiazol-4-carbonsäure
7-Fluor-1-oxo-1H-pyrimido[6,1-b]benzthiazol-4-carbonsäure
8-Trifluormethyl-1-oxo-1H-pyrimido[6,1-b]benzthiazol-4-carbonsäure
N-(5-Tetrazolyl)-7,8-dimethyl-1-oxo-1H-pyrimido[6,1-b]benzthiazol-4-carboxamid
N-(5-Tetrazolyl)-7-methyl-1-oxo-1H-pyrimido[6,1-b]benzthiazol-4-carboxamid
N-(5-Tetrazolyl)-7-hydroxy-1-oxo-1H-pyrimido[6,1-b]benzthiazol-4-carboxamid
N-(5-Tetrazolyl)-7-ethoxy-1-oxo-1H-pyrimido[6,1-b]benzthiazol-4-carboxamid
N-(5-Tetrazolyl)-7,8-methylen-dioxy-1-oxo-1H-pyrimido[6,1-b]benzthiazol-4-carboxamid
N-(5-Tetrazolyl)-7-chlor-1-oxo-1H-pyrimido[6,1-b]benzthiazol-4-carboxamid
N-(5-Tetrazolyl)-8-methoxy-1-oxo-1H-pyrimido[6,1-b]benzthiazol-4-carboxamid
N-(5-Tetrazolyl)-6,8-dimethyl-7-methoxy-1-oxo-1H-pyrimido[6,1-b]benzthiazol-4-carboxamid
N-(5-Tetrazolyl)-7,8-dimethoxy-1-oxo-1H-pyrimido[6,1-b]benzthiazol-4-carboxamid
N-(5-Tetrazolyl)-7-methylmercapto-1-oxo-1H-pyrimido[6,1-b]benzthiazol-4-carboxamid
N-(5-Tetrazolyl)-7-isopropyl-1-oxo-1H-pyrimido[6,1-b]benzthiazol-4-carboxamid
N-(5-Tetrazolyl)-7-t-butyl-1-oxo-1H-pyrimido[6,1-b]benzthiazol-4-carboxamid
N-(5-Tetrazolyl)-7-fluor-1-oxo-1H-pyrimido[6,1-b]benzthiazol-4-carboxamid
N-(5-Tetrazolyl)-8-trifluormethyl-1-oxo-1H-pyrimido[6,1-b]benzthiazol-4-carboxamid
N-(5-Tetrazolyl)-8-nitro-1-oxo-1H-pyrimido[6,1-b]benzthiazol-4-carboxamid

Die nachstehenden Beispiele sollen die Erfindung näher erläutern, ohne sie jedoch zu beschränken.

Die Struktur der in den folgenden Beispielen beschriebenen Verbindungen ist durch CHNS-Analyse, IR-, UV-, NMR- und Massenspektrum gesichert. Einige charakteristische physikalische Daten sind in den einzelnen Beispielen aufgeführt.

Obwohl es in den meisten Fällen nicht notwendig erscheint, bestimmte Zwischenprodukte zu isolieren, werden in den folgenden Beispielen zur zusätzlichen Charakterisierung der Struktur der Endprodukte der allgemeinen Formel I solche Verbindungen mit ihren physikalischen Daten offenbart.

### Beispiel 1

7-Methoxy-1-oxo-1H-pyrimido[6,1-b]benzthiazol-4-carbonsäure

A 1-(2-Mercapto-4-methoxyphenyl)-5-carboxy-pyrimidin-2,6-dion

Variante I

4,5 g 8-Methoxy-4-oxo-4H-pyrimido[2,1-b]benzthiazol-3-carbonsäure-ethylester, hergestellt nach Alaimo (J. Het. Chem. 10, 769 [1973]), werden mit einem Gemisch aus 75 ml 10%iger Natronlauge und 25 ml Ethanol versetzt und 30 Minuten unter Rückfluß erhitzt. Nach Neutralisation mit 2 N-Salzsäure fällt das 1-(2-Mercapto-4-methoxyphenyl)-5-carboxy-pyrimidin-2,6-dion aus.

Ausbeute 3,5 g (80% d. Th.); Fp. 266—267° C (Z)

UV-Spektrum:
pH 7 $\lambda_{max}$: 268 mμ
pH 1 $\lambda_{max}$: 274 mμ
pH 13 $\lambda_{max}$: 293 mμ

Massen-Spektrum: $M^+$ 294, $\frac{m}{e}$ 261

Variante II

Zu einer auf 70° C erwärmten Lösung aus 36 g Natriumhydroxid, 360 ml Wasser und 120 ml Ethanol werden 25 g N-(6-Methoxy-benzthiazol-2-yl)-aminomethylen-malonsäure-diethylester eingetragen und 30 Minuten unter Rückfluß erhitzt. Einen Teil des Ethanols zieht man im Vakuum ab, filtriert und säuert nach dem Abkühlen mit 2 N-Salzsäure an. Man erhält 1-(2-Mercapto-4-methoxyphenyl)-5-carboxy-pyrimidin-2,6-dion in einer Ausbeute von 14,7 g (70% d. Th.). Das Produkt ist nach Schmelzpunkt, IR-, UV- und NMR-Spektrum mit der nach Variante I hergestellten Verbindung identisch.

B Ringschluß zur Titelverbindung
7-Methoxy-1-oxo-1H-pyrimido[6,1-b]benzthiazol-4-carbonsäure

Variante I

111,8 g 1-(2-Mercapto-4-methoxyphenyl)-5-carboxy-pyrimidin-2,6-dion werden durch vierstündiges Erhitzen auf 130° C mit 230 g Polyphosphorsäure unter Wasserabspaltung zu der gewünschten Titelverbindung cyclisiert. Das Reaktionsgemisch wird mit Eis versetzt. Der Niederschlag wird abgesaugt und in Natronlauge aufgenommen. Der Lösung wird anschließend Aktivkohle zugegeben. Danach wird über ein Celite-Filter abgesaugt und mit 2 N-Salzsäure versetzt. Die ausgefällte Säure saugt man ab und erhält nach dem Trocknen 82,5 g (78,6% d. Th.) 7-Methoxy-1-oxo-1H-pyrimido[6,1-b]benzthiazol-4-carbonsäure. Schmelzpunkt 277—278° C (Z). Die Struktur wird durch C,H,N,S-Analyse und spektrale Daten für IR, UV, Massenspektrum und NMR gesichert.

Zum Beispiel Massenspektrum: $M^+$ 276, $\frac{m}{e}$ 232, 205, 217, 190

UV-Spektrum:
pH 7 $\lambda_{max}$: 359 mμ
pH 1 $\lambda_{max}$: 361 mμ
pH 13 $\lambda_{max}$: 359 mμ

Variante II

29,4 g 1-(2-Mercapto-4-methoxyphenyl)-5-carboxy-pyrimidin-2,6-dion werden in 300 ml halbkonzentrierter dioxanischer Salzsäure 6 Stunden zum Sieden erhitzt. Die dabei entstandene 7-Methoxy-1-oxo-1H-pyrimido[6,1-b]benzthiazol-4-carbonsäure kann durch Absaugen der heißen Lösung in praktisch quantitativer Ausbeute gewonnen werden. Die physikalischen Daten stimmen mit der nach Variante I hergestellten Verbindung überein.

Variante III

117,7 g 1-(2-Mercapto-4-methoxyphenyl)-5-carboxy-pyrimidin-2,6-dion werden in 2,3 l Dimethylformamid gelöst und mit 25 ml Amberlyst 15 versetzt. Nach fünfstündigem Rühren bei Raumtemperatur gibt man dem Reaktionsgemisch nochmals 25 ml Amberlyst 15 zu und läßt über Nacht ausreagieren. Der Niederschlag wird abfiltriert und mit 2 N-Kalilauge behandelt. Den Ionenaustauscher saugt man ab und fällt die 7-Methoxy-1-oxo-1H-pyrimido[6,1-b]benzthiazol-4-carbonsäure durch Zugabe von Salzsäure aus.

Ausbeute: 102,8 g (93% d. Th.); Fp. 276—278° C (Z)

In analoger Weise verläuft der Ringschluß mit dem Ionenaustauscher Amberlite JRC 120.

C Herstellung eines Salzes

Die durch Ringschlußreaktion erhaltene 7-Methoxy-1-oxo-1H-pyrimido[6,1-b]benzthiazol-4-carbonsäure wird mit einem geringen Unterschuß wäßriger Natronlauge versetzt. Man filtriert und gewinnt durch Gefriertrocknung das Natriumsalz der 7-Methoxy-1-oxo-1H-pyrimido[6,1-b]benzthiazol-4-carbonsäure in praktisch quantitativer Ausbeute. Wassergehalt: 10,5%; Fp. > 300° C.

Beispiel 2

7-Methoxy-1-oxo-1H-pyrimido[6,1-b]benzthiazol-4-carbonsäure

36 g 6-Methoxy-2-aminobenzthiazol (0,2 mol) werden mit 43,3 g Ethoxymethylen-malonsäure-diethylester (0,2 mol) vereinigt. Es entsteht bei 110°C Badtemperatur eine Lösung. Man erwärmt langsam auf 180° C und destilliert gleichzeitig den entstehenden Alkohol ab. Nach 20 Minuten versetzt man die erkaltete Schmelze mit 320 ml Ethanol und einer Lösung von 100 g Natriumhydroxid in 1000 ml Wasser und erwärmt das Gemisch 1 Stunde unter Rückfluß. Im Vakuum wird der größte Teil des Alkohols entfernt und die wäßrige Lösung des Zwischenprodukts mit 500 ml konzentrierter Salzsäure versetzt. Anschließend destilliert man Wasser ab, bis die Destillationstemperatur 108°C erreicht

(20%ige Salzsäure), kühlt ab und isoliert den ausgefallenen Niederschlag, der mit Wasser nachgewaschen und bei 70°C getrocknet wird. Die gewünschte 7-Methoxy-1-oxo-1H-pyrimido[6,1-b]benzthiazol-4-carbonsäure wird in einer Ausbeute von 47,9 g (86,7% d. Th.) erhalten. Der Schmelzpunkt von 278—280°C und das IR-Spektrum beweisen die Identität mit der nach dem Verfahren des Beispiels 1B erhaltenen Verbindung.

## Beispiel 3

### 8-Methoxy-1-oxo-1H-pyrimido[6,1-b]benzthiazol-4-carbonsäure

N-(5-Methoxy-benzthiazol-2-yl)-aminomethylen-malonsäure-diethylester wird, wie in Beispiel 1A, Variante II beschrieben, in einem Gemisch aus 10%iger Natronlauge und Ethanol erhitzt. Das Reaktionsgemisch wird, ohne das als Zwischenprodukt auftretende 1-(2-Mercapto-5-methoxy-phenyl)-5-carboxy-pyrimidin-2,6-dion zu isolieren, stark angesäuert. Dabei fällt die 8-Methoxy-1-oxo-1H-pyrimido[6,1-b]benzthiazol-4-carbonsäure aus. Der Niederschlag wird ungefähr 1 Stunde im sauren Milieu nachgerührt.

Fp. 277—278°C.

Massenspektrum: $M^+$ 276, $\frac{m}{e}$ 232, 205, 190

UV-Spektrum:
pH 1 $\lambda_{max}$: 368 mμ
pH 13 $\lambda_{max}$: 365 mμ

## Beispiel 4

### 7-Methoxy-1-oxo-1H-pyrimido[6,1-b]benzthiazol-4-carbonsäure-ethylester

20 g des gemäß Beispiel 1C hergestellten Natriumsalzes der 7-Methoxy-1-oxo-1H-pyrimido[6,1-b]-benzthiazol-4-carbonsäure werden in 200 ml Hexametapol aufgeschlämmt und bei Raumtemperatur mit 23,45 g Ethyliodid versetzt. Man rührt 80 Minuten und gießt die entstandene Lösung in 3 l Wasser. Der Niederschlag wird noch feucht mit 500 ml Ethanol aufgekocht und durch Zusatz von etwas Nitromethan in Lösung gebracht. Nach dem Abkühlen kristallisieren 12,9 g (63,3% d. Th.) 7-Methoxy-1-oxo-1H-pyrimido[6,1-b]benzthiazol-4-carbonsäure-ethylester mit einem Schmelzpunkt von 231—232°C aus.

In analoger Weise erhält man aus dem Natriumsalz der 7-Methoxy-1-oxo-1H-pyrimido[6,1-b]benzthiazol-4-carbonsäure und Methyliodid 7-Methoxy-1-oxo-1H-pyrimido[6,1-b]benzthiazol-4-carbonsäure-methylester; Fp. 253—254°C.

## Beispiel 5

### 1-Oxo-1H-pyrimido[6,1-b]benzthiazol-4-carbonsäure

A 1-(2-Mercapto-phenyl)-5-carboxy-pyrimidin-2,6-dion

Gemäß Beispiel 1A, Variante I erhält man aus 4-Oxo-4-H-pyrimido[2,1-b]benzthiazol-carbonsäure das 1-(2-Mercapto-phenyl)-5-carboxy-pyrimidin-2,6-dion in 77,7%iger Ausbeute. Fp. 254—255°C. Nach Variante II des Beispiels 1A wird aus Benzthiazol-2-yl-aminomethylen-malonsäurediethylester die gleiche Verbindung in 52%iger Ausbeute gewonnen. Fp. 254—255°C.

Massenspektrum: $M^+$ 264, $\frac{m}{e}$ 231, 187, 151

UV (Chloroform/MeOH):
$\lambda_{max}$: 277 mμ
pH 1 $\lambda_{max}$: 274 mμ
pH 13 $\lambda_{max}$: 292 mμ

B 1-Oxo-1H-pyrimido[6,1-b]benzthiazol-4-carbonsäure

Gemäß Beispiel 1B, Variante III werden 31,7 g 1-(2-Mercapto-phenyl)-5-carboxy-pyrimidin-2,6-dion in 310 ml Dimethylformamid nach Zusatz von insgesamt 40 ml Amberlite Amberlyst 15 12 Stunden bei Raumtemperatur gerührt. Nach der im Beispiel 1B, Variante III angegebenen Aufarbeitung erhält man 20,8 g 1-Oxo-1H-pyrimido[6,1-b]benzthiazol-4-carbonsäure (70,4% d. Th.) vom Schmelzpunkt 278—279° C (Z).

Massenspektrum: $M^+$ 246, $\frac{m}{e}$ 202, 175, 160

NMR (DDMSO): 3 H, 8,86 ppm

UV-Spektrum:
pH 7 $\lambda_{max}$: 356 mμ
pH 13 $\lambda_{max}$: 358 mμ

Der Ringschluß kann auch durch Erhitzen mit 20%iger Salzsäure durchgeführt werden.

C Gemäß Beispiel 1C wird ein Natriumsalz (Wassergehalt 8%) hergestellt; Fp. > 300° C.

In analoger Weise erhält man

a) aus 8-Ethoxy-4-oxo-4H-pyrimido[2,1-b]benzthiazol-3-carbonsäure-ethylester über das 1-(2-Mercapto-4-ethoxyphenyl)-5-carboxy-pyrimidin-2,6-dion (Ausbeute: 86,9% d. Th.; Fp. 273—275° C) die 7-Ethoxy-1-oxo-1H-pyrimido[6,1-b]benzthiazol-4-carbonsäure (Ausbeute: 74,5% d. Th.; Fp. 264—265° C)

UV-Spektrum:
pH 7 $\lambda_{max}$: 359 mμ
pH 1 $\lambda_{max}$: 361 mμ
pH 13 $\lambda_{max}$: 359 mμ

Massenspektrum: $M^+$ 290, $\frac{m}{e}$ 246, 219, 204

Durch Zugabe von 1 N-Kalilauge in geringem Unterschuß und anschließender Gefriertrocknung erhält man das Kaliumsalz der 7-Ethoxy-1-oxo-1H-pyrimido[6,1-b]benzthiazol-4-carbonsäure (Wassergehalt 5,6%). Fp. > 300° C

b) aus 8-Hydroxy-4-oxo-4H-pyrimido[2,1-b]benzthiazol-3-carbonsäureethylester über das 1-(2-Mercapto-4-hydroxyphenyl)-5-carboxy-pyrimidin-2,6-dion (Ausbeute 60,7% d. Th.; Fp. 230—232° C) die 7-Hydroxy-1-oxo-1H-pyrimido[6,1-b]benzthiazol-4-carbonsäure (Ausbeute 51,1% d. Th.; Fp. 280—281° C).

Massenspektrum: Trimethylsilyl-Derivat $M^+$ 406, $\frac{m}{e}$ 391, 364, 263, 188, 153

UV-Spektrum:
pH 7 $\lambda_{max}$: 360 mμ
pH 1 $\lambda_{max}$: 364 mμ
pH 13 $\lambda_{max}$: 376 mμ

Zur Herstellung des Kaliumsalzes wird die Säure mit einem geringen Unterschuß wäßriger Kaliumbicarbonat-Lösung versetzt. Man filtriert und isoliert das Kaliumsalz durch Gefriertrocknung; Fp. > 300° C.

c) aus 7,8-Methylendioxy-4-oxo-4H-pyrimido[2,1-b]benzthiazol-3-carbonsäureethylester über das 1-(2-Mercapto-4,5-methylendioxy-phenyl)-5-carboxy-pyrimidin-2,6-dion (Ausbeute 70,3% d. Th.; Fp. 273—275° C) die 7,8-Methylendioxy-1-oxo-1H-pyrimido[6,1-b]benzthiazol-4-carbonsäure (Ausbeute 71,7% d. Th.; Fp. 283—284° C (Z))

Massenspektrum: $M^+$ 290, $\frac{m}{e}$ 246, 219, 204

d) aus 7,8-Dimethyl-4-oxo-4H-pyrimido[2,1-b]benzthiazol-3-carbonsäure-ethylester über das 1-(2-Mercapto-4,5-dimethyl-phenyl)-5-carboxy-pyrimidin-2,6-dion (Ausbeute 89,7% d. Th.; Fp.

258—259° C (Z)); Massenspektrum: M+ 292, $\frac{m}{e}$ 259, 215, 179, 151; die 7,8-Dimethyl-1-oxo-1H-pyrimido[6,1-b]benzthiazol-4-carbonsäure (Ausbeute 56,2% d. Th.; Fp. 280—281° C)

Massenspektrum: M+ 274, $\frac{m}{e}$ 230, 203, 188

NMR (DDMSO): 3 H, 8,81 ppm

UV-Spektrum:
pH 1 $\lambda_{max}$: 363 mμ
pH 13 $\lambda_{max}$: 361 mμ

e) aus 8-Methyl-4-oxo-4H-pyrimido[2,1-b]benzthiazol-3-carbonsäureethylester über das 1-(2-Mercapto-4-methylphenyl)-5-carboxy-pyrimidin-2,6-dion (Ausbeute 77,7% d. Th.; Fp. 258—260° C) die 7-Methyl-1-oxo-1H-pyrimido[6,1-b]benzthiazol-4-carbonsäure (Ausbeute 63% d. Th.; Fp. 263—264° C)

Massenspektrum: M+ 260, $\frac{m}{e}$ 216, 189, 174

NMR (DDMSO): 3 H, 8,83 ppm

UV (DMF + MeOH):
$\lambda_{max}$: 360 mμ
pH 7 $\lambda_{max}$: 360 mμ
pH 1 $\lambda_{max}$: 354 mμ
pH 13 $\lambda_{max}$: 359— mμ

f) aus 8-Chlor-4-oxo-4H-pyrimido[2,1-b]benzthiazol-3-carbonsäureethylester über das 1-(2-Mercapto-4-chlorphenyl)-5-carboxy-pyrimidin-2,6-dion die 7-Chlor-1-oxo-1H-pyrimido[6,1-b]benzthiazol-4-carbonsäure (Ausbeute 60% d. Th.; Fp. 264—265° C)

Massenspektrum: M+ 280, $\frac{m}{e}$ 236, 209, 194

g) aus 7,9-Dimethyl-8-methoxy-4-oxo-4H-pyrimido[2,1-b]benzthiazol-3-carbonsäure-methylester (Fp. 146—148° C) über das 1-(2-Mercapto-3,5-dimethyl-4-methoxy-phenyl)-5-carboxy-pyrimidin-2,6-dion (quantitative Ausbeute, Fp. 249—150° C; UV (MeOH): $\lambda_{max}$: 273 mμ; pH 1 $\lambda_{max}$: 271 mμ; pH 13 $\lambda_{max}$: 291 mμ) die 6,8-Dimethyl-7-methoxy-1-oxo-1H-pyrimido[6,1-b]benzthiazol-4-carbonsäure (Ausbeute 58,8% d. Th.; Fp. 269—272° C)

Massenspektrum: M+ 304, $\frac{m}{e}$ 260, 245, 233, 218, 203

UV (MeOH + DMF):
$\lambda_{max}$: 366 mμ
pH 1 $\lambda_{max}$: 365 mμ
pH 13 $\lambda_{max}$: 364 mμ

h) aus 7,8-Dimethoxy-4-oxo-4H-pyrimido[2,1-b]benzthiazol-3-carbonsäure-ethylester über das 1-(2-Mercapto-4,5-dimethoxy-phenyl)-5-carboxy-pyrimidin-2,6-dion (quantitative Ausbeute) die 7,8-Dimethoxy-1-oxo-1H-pyrimido[6,1-b]benzthiazol-4-carbonsäure (Ausbeute 53,9% d. Th.; Fp. 260—261° C (Z))

Massenspektrum: M+ 306

Kaliumsalz: UV ($H_2O$):
$\lambda_{max}$: 371 mμ
pH 1 $\lambda_{max}$: 388 mμ
pH 13 $\lambda_{max}$: 369 mμ
pH 7 $\lambda_{max}$: 371 mμ

i) aus 8-Methylmercapto-4-oxo-4H-pyrimido[2,1-b]benzthiazol-3-carbonsäureethylester über das 1-(2-Mercapto-4-methylmercapto-phenyl)-5-carboxy-pyrimidin-2,6-dion (quantitative Ausbeute)

Massenspektrum: M+ 310, 292

UV (MeOH):
$\lambda_{max}$: 262 mμ
pH 1 $\lambda_{max}$: 265 mμ
pH 13 $\lambda_{max}$: 276 mμ

die 7-Methylmercapto-1-oxo-1H-pyrimido[6,1-b]benzthiazol-4-carbonsäure (Ausbeute 73% d. Th.; Fp. 260—261°C)

Massenspektrum: M+292

UV-Spektrum:
pH 13 $\lambda_{max}$: 363 mμ
pH 1 $\lambda_{max}$: 369 mμ

j) aus 8-Isopropyl-4-oxo-4H-pyrimido[2,1-b]benzthiazol-3-carbonsäureethylester über das 1-(2-Mercapto-4-isopropylphenyl)-5-carboxy-pyrimidin-2,6-dion die 7-Isopropyl-1-oxo-1H-pyrimido[6,1-b]benzthiazol-4-carbonsäure: Fp. 240—241°C.

Beispiel 6

8-Nitro-1-oxo-1H-pyrimido[6,1-b]benzthiazol-4-carbonsäure

5,52 g 1-Oxo-1H-pyrimido[6,1-b]benzthiazol-4-carbonsäure (hergestellt nach Beispiel 5) werden zu einem Gemisch von 27,5 ml konzentrierter Schwefelsäure und 27,5 ml 96%iger Salpetersäure bei 0—5°C eingetragen. Man rührt eine Stunde bei 0°C und 15 Minuten bei Raumtemperatur nach und gießt den Ansatz auf Eis. Der ausgefallene Niederschlag wird anschließend aus einem Gemisch Ethanol/Dimethylformamid umkristallisiert. Es verbleiben 3,6 g 8-Nitro-1-oxo-1H-pyrimido[6,1-b]benzthiazol-4-carbonsäure (62,1% d. Th.) vom Schmelzpunkt 277—278°C.

Gemäß Beispiel 1 C wird mit 1 N Kalilauge neutralisiert und durch Gefriertrocknung das entsprechende Kaliumsalz isoliert (6% Wassergehalt).

Beispiel 7

N-(5-Tetrazolyl)-7-methoxy-1-oxo-1H-pyrimido[6,1-b]benzthiazol-4-carboxamid

5,52 g 7-Methoxy-1-oxo-1H-pyrimido[6,1-b]benzthiazol-4-carbonsäure (hergestellt nach Beispiel 1) werden in 150 ml Dimethylformamid mit 7,14 g N,N'-Carbonyldiimidazol vereinigt. Anschließend rührt man das Gemisch 90 Minuten bei 100°C und 1 Stunde bei Raumtemperatur, versetzt dann mit 2,26 g 5-Aminotetrazol-monohydrat und erhitzt wieder 3 Stunden auf 100°C. Das Lösungsmittel entfernt man weitgehend im Vakuum und rührt den Rückstand mit Wasser aus. Der Niederschlag besteht aus 5,2 g N-(5-Tetrazolyl)-7-methoxy-1-oxo-1H-pyrimido[6,1-b]benzthiazol-4-carboxamid (75,7% d. Th.); Fp. 234—235°C (aus Nitromethan).

Titration in Dimethylformamid mit 0,1 N Tetrabutylammoniumhydroxid: Äquivalentgewicht 343.

Durch Neutralisation mit 1 N Kalilauge und anschließender Gefriertrocknung wird das entsprechende Kaliumsalz isoliert.

UV ($H_2O$):
$\lambda_{max}$: 346 mμ
pH 1 $\lambda_{max}$: 357 mμ
pH 13 $\lambda_{max}$: 346 mμ

Beispiel 8

N-(5-Tetrazolyl)-1-oxo-1H-pyrimido[6,1-b]benzthiazol-4-carboxamid

In analoger Weise, wie in Beispiel 7 beschrieben, erhält man aus 1-Oxo-1H-pyrimido[6,1-b]benzthiazol-4-carbonsäure (hergestellt nach Beispiel 5) (4,9 g), N,N'-Carbonyldiimidazol (3,6 g) und wasserfreiem Aminotetrazol (1,9 g) das N-(5-Tetrazolyl)-1-oxo-1H-pyrimido[6,1-b]benzthiazol-carboxamid.

Ausbeute: 80,3% d. Th.; Fp. 243—244°C.

Durch Titration mit 1 N Kalilauge und anschließender Gefriertrocknung der wäßrigen Lösung wird das entsprechende Kaliumsalz hergestellt.

## Patentansprüche

1. 1-Oxo-1H-pyrimido(6,1-b)benzthiazole der allgemeinen Formel I

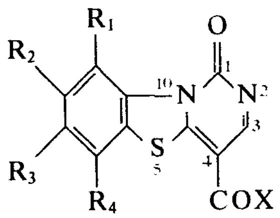

(I)

in der

$R_1$, $R_2$, $R_3$ und $R_4$ jeweils Wasserstoff oder Halogen, eine Hydroxy-, Nitro- oder Trifluormethyl-Gruppe, einen niederen geradkettigen oder verzweigten Alkyl-, Alkoxy- oder Alkylthio-Rest bedeuten, wobei $R_2$ und $R_3$ zusammen auch eine Alkylendioxy-Gruppe vorstellen können, und
X eine Hydroxy-, Alkoxy- oder Tetrazolyl-5-amino-Gruppe bedeutet,
und deren pharmakologisch verträgliche Salze.

2. Verbindungen der Formel I gemäß Anspruch 1, in der $R_1$ Wasserstoff und X Hydroxy darstellen und $R_2$, $R_3$ und $R_4$ jeweils die angegebene Bedeutung haben.

3. Verbindungen der Formel I gemäß Anspruch 1, in der $R_1$ Wasserstoff und X eine Tetrazolyl-5-amino-Gruppe darstellen und $R_2$, $R_3$ und $R_4$ die angegebene Bedeutung haben.

4. Verfahren zur Herstellung von Verbindungen gemäß Anspruch 1 bis 3, mit den angegebenen Bedeutungen der Substituenten und deren pharmakologisch verträglichen Salzen, dadurch gekennzeichnet, daß man ein 4-Oxo-4H-pyrimido(2,1-b)benzthiazol der allgemeinen Formel II

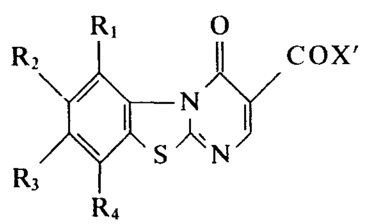

(II)

in der

$R_1$, $R_2$, $R_3$, $R_4$ die oben angegebene Bedeutung haben und
X' einen niederen Alkoxyrest oder eine Hydroxygruppe vorstellt,

mit überschüssiger Base zu den in Lösung im tautomeren Gleichgewicht stehenden 1-(2-Mercaptophenyl)-pyrimidin-2,6-dionen der allgemeinen Formel III A—III C

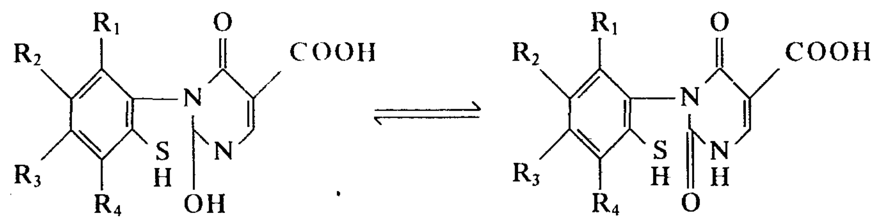

(IIIA) (IIIB)

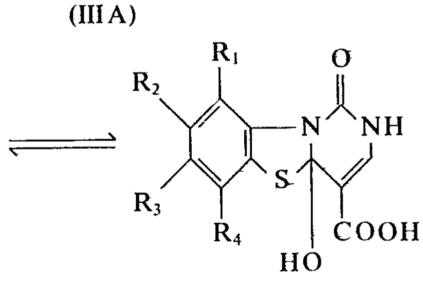

(IIIC)

in denen

$R_1$, $R_2$, $R_3$ und $R_4$ die oben angegebene Bedeutung haben,
bzw. deren Alkalisalzen hydrolysiert und diese unter sauren Reaktionsbedingungen durch Wasserabspaltung zu erfindungsgemäßen Verbindungen der allgemeinen Formel I, in der X eine Hydroxygruppe bedeutet, cyclisiert,
wobei man gegebenenfalls anschließend in beliebiger Reihenfolge
die erhaltenen Carbonsäuren in an sich bekannter Weise in deren Ester oder Tetrazolyl-5-amide der allgemeinen Formel I, in der X eine Alkoxy- oder Tetrazolyl-5-amino-Gruppe vorstellt, überführt
für den Fall, daß in Verbindungen der allgemeinen Formel I einer der Reste $R_1$, $R_2$, $R_3$ und $R_4$ eine Nitrogruppe bedeutet, diese gegebenenfalls nachträglich einführt, einen bestimmten Substituenten $R_1$, $R_2$, $R_3$, $R_4$ oder X in einen anderen Substituenten $R_1$, $R_2$, $R_3$, $R_4$ oder X umwandelt
und/oder die erhaltenen Carbonsäuren bzw. Tetrazolylamide der Formel I in pharmakologisch verträgliche Salze überführt.

5. Abwandlung des Verfahrens gemäß Anspruch 4 dadurch gekennzeichnet, daß man Aminomethylenverbindungen der allgemeinen Formel V

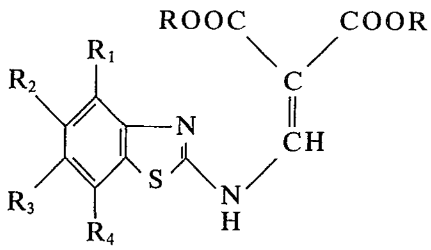

(V)

in der

$R_1$, $R_2$, $R_3$ und $R_4$ die im Anspruch 4 angegebene Bedeutung haben
und R eine niedere Alkylgruppe vorstellt,
in überschüssigen Basen zu den Zwischenprodukten der allgemeinen Formel III umsetzt.

6. Verbindungen gemäß Anspruch 1 bis 3 zur Verwendung bei der Bekämpfung allergischer Krankheiten.

7. Arzneimittel, gekennzeichnet durch einen Gehalt an Verbindungen gemäß Anspruch 1 bis 3 und üblichen Träger- und Hilfsstoffen.

**Claims**

1. 1-Oxo-1H-pyrimido(6,1-b)benzthiazoles of the general formula I

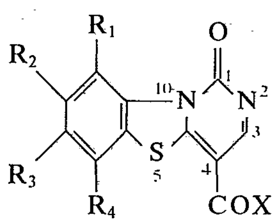

(I)

in which $R_1$, $R_2$, $R_3$ and $R_4$ each signify hydrogen or halogen, a hydroxyl, nitro or trifluormethyl group, a lower straight-chained or branched alkyl, alkoxy or alkylthio radical, whereby $R_2$ and $R_3$ can together also represent an alkylenedioxy group, and X signifies a hydroxyl, alkoxy or tetrazolyl-5-amino group; and the pharmacologically acceptable salts thereof.

2. Compounds of formula I according to claim 1, in which $R_1$ signifies hydrogen and X hydroxyl and $R_2$, $R_3$ and $R_4$ each have the given meaning.

3. Compounds of formula I according to claim 1, in which $R_1$ signifies hydrogen and X a tetrazolyl-5-amino group and $R_2$, $R_3$ and $R_4$ have the given meaning.

4. Process for the preparation of compounds according to claims 1 to 3 with the given meanings of the substituents and of their pharmacologically acceptable salts, characterised in that one hydrolyses a 4-oxo-4H-pyrimido(2,1-b)benzthiazole of the general formula II

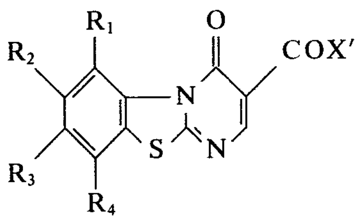

(II)

in which $R_1$, $R_2$, $R_3$, $R_4$ have the above-given meaning and X' represents a lower alkoxy radical or a hydroxyl group, with excess base to give 1-(2-mercaptophenyl)-pyrimidin-2,6-diones of general formulae IIIA—IIIC, which are in tautomeric equilibrium in solution:

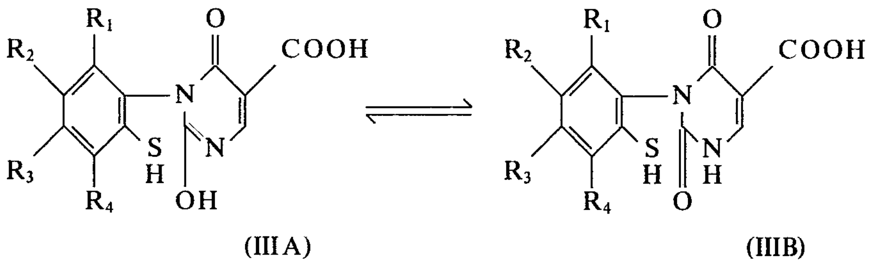

(IIIA) (IIIB)

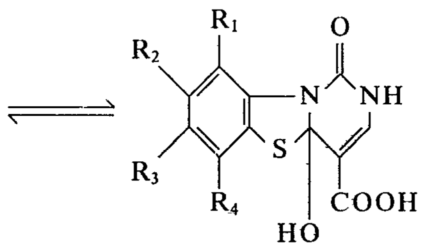

(IIIC)

in which $R_1$, $R_2$, $R_3$ and $R_4$ have the above-given meaning, or their alkali metal salts and these are cyclised under acidic reaction conditions, by the splitting off of water, to give compounds of general formula I according to the invention in which X signifies a hydroxyl group,
whereby, in any desired sequence, one possibly subsequently converts the carboxylic acids obtained in per se known manner into their esters or tetrazolyl-5-amides of the general formula I, in which X represents an alkoxy or tetrazolyl-5-amino group,
for the case in which, in compounds of the general formula I, one of the residues $R_1$, $R_2$, $R_3$ and $R_4$ signifies a nitro group, this is possibly subsequently introduced, a particular substituent $R_1$, $R_2$, $R_3$, $R_4$ or X is converted into another substituent $R_1$, $R_2$, $R_3$, $R_4$ or X
and/or the carboxylic acids or tetrazolylamides obtained of formula I are converted into pharmacologically acceptable salts.

5. Modification of the process according to claim 4, characterised in that one reacts aminomethylene compounds of the general formula V

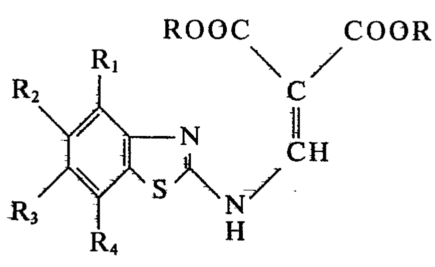

(V)

in which $R_1$, $R_2$, $R_3$ and $R_4$ have the meaning given in claim 4 and R represents a lower alkyl group, in excess bases to give the intermediate products of the general formula III.

6. Compounds according to claim 1 to 3 for use in combating allergic diseases.

7. Medicaments, characterised by a content of compounds according to claim 1 to 3 and conventional carrier and adjuvant materials.

**Revendications**

1. Dérivés de 1-oxo 1H-pyrimido (6,1-b)benzothiazole de formule générale I

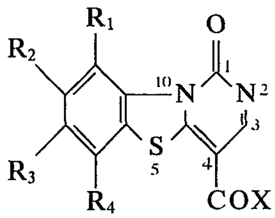

(I)

dans laquelle

$R_1$, $R_2$, $R_3$ et $R_4$ sont chacun un atome d'hydrogène ou d'halogène, un groupe hydroxyle, nitro ou trifluorométhyle, un reste alkyle, alcoxy ou alkylthio inférieur à chaîne droite ou ramifiée, $R_2$ et $R_3$ pouvant représenter aussi ensemble un groupe alkylène-dioxy, et X est un groupe hydroxyle, alcoxy ou tétrazolyl 5-amino,
ainsi que leurs sels pharmacologiquement tolérables.

2. Dérivés de formule I selon la revendication 1, dans laquelle $R_1$ est de l'hydrogène et X un groupe hydroxyle et $R_2$, $R_3$ et $R_4$ ont la signification ci-dessus indiquée.

3. Dérivés de formule I selon la revendication 1 dans laquelle $R_1$ est de l'hydrogène et X un groupe tétrazolyle 5-amino et $R_2$, $R_3$ et $R_4$ ont la signification ci-dessus indiquée.

4. Procédé pour la préparation des dérivés selon les revendications 1 à 3, dans lesquels les substituants sont ceux ci-dessus indiqués, et de leurs sels pharmacologiquement tolétables, caractérisé en ce que l'on soumet un 4-oxo 4H-pyramido (2,1-b)benzothiazole de formule II

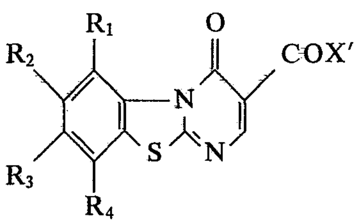

(II)

dans laquelle

$R_1$, $R_2$, $R_3$, $R_4$ ont la signification ci-dessus indiquée et X' est un reste alcoxy inférieur ou un groupe hydroxy, à une hydrolyse en présence d'un excès de base pour former les 1-(2-mercaptophényl)pyrimidine 2,6-diones dont les trois formes tautomères représentées par les formules IIIA à IIIC ci-après sont en équilibre:

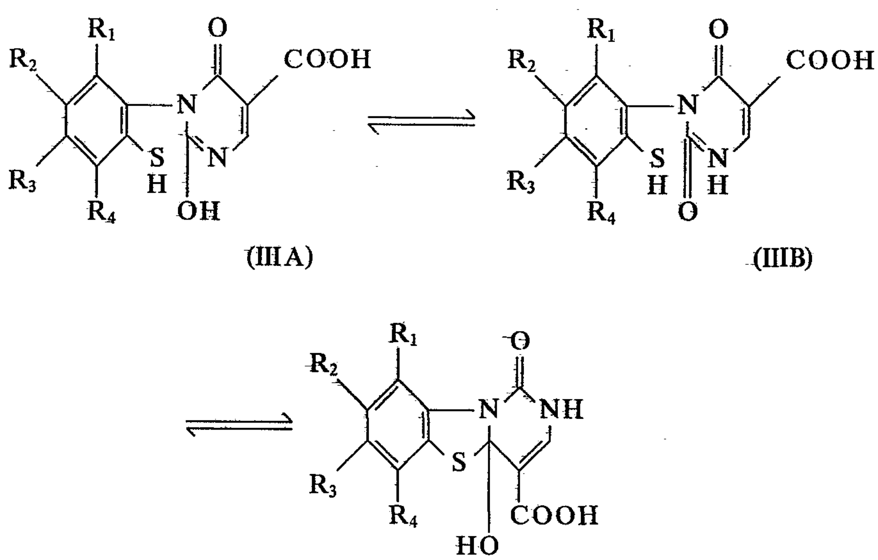

(IIIA) (IIIB)

(IIIC)

où $R_1$, $R_2$, $R_3$ et $R_4$ ont la signification ci-dessus indiquée, ainsi que leurs sels alcalins, et en ce que ces produits obtenus sont cyclisés en milieu acide par élimination d'eau pour obtenir les composés de formule I selon l'invention, dans lesquels X est un groupe hydroxyle, et en ce qu'éventuellement ensuite, dans un ordre quelconque: on transforme les acides carboxyliques obtenus de façon en soi connue en leurs ester ou leurs tétrazolyl 5-amides de formule générale I dans laquelle X est un groupe alcoxy ou tétrazolyl 5-amino; dans le cas où dans des composés de formule générale I un des restes $R_1$, $R_2$ $R_3$ ou $R_4$ est un groupe nitro, on introduit celui-ci éventuellement ultérieurement; on transforme un substituant $R_1$, $R_2$, $R_3$, $R_4$ ou X en un autre substituant $R_1$, $R_2$, $R_3$, $R_4$ ou x; et/ou on transforme les acides carboxyliques ou les tétrazolylamides de formule I obtenus en leurs sels pharmacologiquement tolérables.

5. Procédé modifié selon la revendication 4 caractérisé en ce que l'on fait réagir des dérivés d'aminométhylène de formule générale V

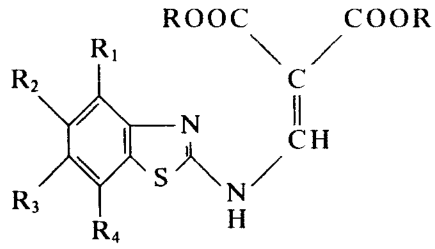

(V)

dans laquelle

$R_1$, $R_2$, $R_3$ et $R_4$ ont la signification donnée dans la revendication 4, et R est un groupe alkyleinférieur, dans un excès de base pour obtenir les produits intermédiaires de formule générale III.

6. Dérivés selon l'une des revendications 1 à 3 pour la lutte contre les maladies allergiques.

7. Médicaments caractérisés en ce qu'ils ont une teneur en composés selon l'une des revendications 1 à 3 et en substance de support et auxiliaires.